# FASCICULE DE BREVET EUROPEEN

(11) **EP 0 820 434 B1**
(45) Date de publication et mention de la délivrance du brevet: **09.08.2000**
(21) Numéro de dépôt: 96912070.8
(22) Date de dépôt: 05.04.1996
(51) Int. Cl.: C07C 233/78, C07C 233/62, C07D 213/81, C07C 237/42, C07C 255/54, C07C 323/20, C07C 235/50, C07C 235/42, C07C 235/46, C07C 233/36, C07C 259/10

(54) **DERIVES DE LA PHENOXYETHYLAMINE POSSEDANT UNE HAUTE AFFINITE POUR LE RECEPTEUR 5-HT 1A, LEUR PROCEDE DE PREPARATION, LEUR APPLICATION COMME MEDICAMENT ET LES COMPOSITIONS PHARMACEUTIQUES LES RENFERMANT**
PHENOXYETHYLAMIN-DERIVATE MIT HOHER AFFINITÄT FUR DEN 5-HT1A-REZEPTOR, VERFAHREN ZU IHRER HERSTELLUNG, IHRE VERWENDUNG ALS MEDIKAMENTE UND SIE ENTHALTENDE PHARMAZEUTISCHE ZUSAMMENSETZUNGEN
PHENOXYETHYLAMINE DERIVATIVES HAVING HIGH AFFINITY FOR THE 5-HT 1A RECEPTOR, PREPARATION THEREOF, USE THEREOF AS DRUGS, AND PHARMACEUTICAL COMPOSITIONS CONTAINING SAID DERIVATIVES

(30) Priorité: 07.04.1995 GB 9507288
(43) Date de publication de la demande: 28.01.1998
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif-sur-Yvette (FR); POMMIER, Jacques, F-92700 Colombes (FR); MARTIN, Christiane, F-92350 Le Plessis-Robinson (FR); ROUBERT, Pierre, F-75014 Paris (FR); DEFAUX, Jean-Pierre, F-92500 Rueil-Malmaison (FR)
(74) Mandataire: Bourgouin, André
(86) Numéro de dépôt international: FR9600518
(87) Numéro de publication internationale: WO9631461

(56) Documents cités:
- EP-A- 0 558 245
- WO-A-95/05366
- DRUG DEVELOPMENT RESEARCH, vol. 26, no. 3, 1992, pages 251-274, XP002008941 R. A. GLENNON: "Concepts for the design of 5-HT1a seretonin agonists and antagonists"
- EXPERT OPINION ON THERAPEUTIC PATENTS, vol. 4, no. 10, 1994, pages 1233-1241, XP002008942 G. M. SHUTSKE: "Recent patent activity relating to serotonin pharmacology"

## Description

Le neurotransmetteur sérotonine (5-hydroxytryptamine, 5-HT) a une grande importance dans l'étiologie et dans le traitement de très nombreux problèmes médicaux [R.A. Glennon, J. Med. Chem., 30, 1 (1987)]. De multiples récepteurs 5-HT ont désormais été identifiés et représentent des objectifs intéressants pour la manipulation sélective de la fonction sérotoninergique. Le sous-type 5-HT_{1A} est particulièrement intéressant dans la mesure où les composés agissant sur ce récepteur peuvent être utilisés dans le traitement de l'anxiété, de la dépression, des troubles du sommeil ou des problèmes cardiovasculaires [Brain 5-HT_{1A} Receptors : Behavioural and Neurochemical Pharmacology ; Editors C.T. Dourish, S. Ahlenius, P.H. Huston ; Ellis Horwood Ltd. ; Chichester (1987)]. Les agonistes 5-HT_{1A} peuvent également être utilisés en tant que anti-émétiques [U. Wells, M. Ravenscroft, P. Bhandari, P.L.R. Andrews, Med. Sci. Symp. Ser., 5, 179 (1993)]; F. Okada, Y.Torii, H. Saito, N. Matsuki, Jpn. J. Pharmacol., 64, 109 (1994)] ou en tant qu'agents anti-sécrétoires [J.S. Gidda, J.M. Schaus, EP. 455.510 A2 ; D.C. Evans, J.S. Gidda, Gastroenterology, 104, A76 (1993)].

La présente invention concerne de nouveaux dérivés de phénoxyéthylamine possédant une haute affinité pour le récepteur 5-HT_{1A}, des procédés pour leur préparation, des compositions pharmaceutiques les contenant et leur utilisation notamment en tant qu'inhibiteurs de sécrétion d'acide gastrique ou en tant qu'anti-émétiques.

L'invention a ainsi pour objet les produits de formule générale I 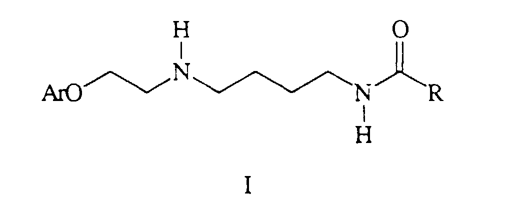 dans laquelle
- Ar: représente un phényle substitué par un ou plusieurs substituants choisis parmi les radicaux halogène, alkyle ayant de 1 à 6 atomes de carbone, alkoxy ayant de 1 à 6 atomes de carbone, cyano, nitro, hydroxy, C(O)NR¹R², C(O)NHOR³, NHC(O)R⁴, NHC(O)NHR⁵, CH₂NHC(O)NHR⁶, CH₂OR⁷, CH₂NHC(O)R⁸, CO₂R⁹, NHCO₂R¹⁰, C(O)R¹¹, SR¹² ;
dans lesquels R¹, R², R³, R⁴, R⁵, R⁶, R⁷, R⁸ sont, indépendamment, un atome d'hydrogène ou un alkyle ayant de 1 à 6 atomes de carbone et R⁹, R¹⁰, R¹¹, R¹² sont, indépendamment, un alkyle ayant de 1 à 6 atomes de carbone ;
- R: représente un radical hydrocarboné contenant de 1 à 10 atomes de carbone choisi parmi les radicaux alkyle, alkényle, alkynyle linéaires ou ramifiés, les radicaux cycloalkyle, cycloalkylalkyle ou alkylcycloalkyle, monocycliques ou polycycliques, saturés ou insaturés ;
le radical pyridyl ou isoquinolyl,
un phényle, éventuellement substitué par un ou plusieurs substituants choisis parmi les radicaux alkyle ayant de 1 à 6 atomes de carbone, alkoxy ayant de 1 à 6 atomes de carbone, halogène, hydroxy, nitro amino ou acylamino,
ainsi que les sels de ces produits.

Dans les définitions indiquées ci-dessus, l'expression halogène représente un atome de fluor, de chlore, de brome ou d'iode, de préférence fluor ou iode. L'expression alkyle inférieure représente de préférence un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle.

Parmi les radicaux alkényle, on peut citer les radicaux vinyle, allyle, butényle.

Parmi les radicaux alkynyle, on peut citer les radicaux éthynyle ou propargyle.

Parmi les radicaux acylamino, on peut citer les radicaux acétylamino, propionylamino.

Les radicaux alkoxy inférieurs peuvent correspondre aux radicaux alkyle indiqués ci-dessus. On préfère les radicaux méthoxy, éthoxy ou isopropyloxy.

Les radicaux cycloalkyles monocycliques saturés peuvent être choisis parmi les radicaux ayant de 3 à 7 atomes de carbone tels que les radicaux cyclopropyle, cyclobutyle, cyclopentyle, cyclohexyle ou cycloheptyle.

Les radicaux cycloalkyles mono ou polysaturés peuvent être choisis parmi les radicaux cyclobutène, cyclopentène, cyclohexène, cyclopentanediène, cyclohexadiène.

Des exemples de radical cycloalkyle polycyclique sont le bicyclo[2,2,l]heptyle ou l'adamantyle.

Les produits de formule 1 peuvent former des sels d'addition avec les acides, en particulier les acides pharmacologiquement acceptables.

Des exemples de sels sont donnés ci-après dans la partie expérimentale.

L'invention a particulièrement pour objet les composés de formule générale 1 telle que décrite ci-dessus, caractérisée en ce que Ar représente un radical phényle substitué par un substituant choisi parmi les radicaux hydroxy, méthoxy, -C(O)NHMé, -NHC(O)Mé, NHCO₂R'₁₀, R'₁₀ représentant un radical méthyle ou éthyle, -NHCONH₂, -C(O)NHOH et en ce que R représente un radical cyclopentyle, cyclohexyle, cycloheptyle, cyclopentylméthyle, adamantyle, tert-butyle, neopentyle, phényle, fluorophényle.

Les substituants du radical phényle que peut représenter Ar sont de préférence situés en position 2 ou 3.

Plus particulièrement, l'invention a pour objet les produits décrits ci-après dans les exemples, en particulier les produits répondant aux formules suivantes :
- la N[4-{2-(2-méthoxyphénoxy)ethyl}aminobutyl] benzamide;
- la N[4-{2-(2-méthoxyphénoxy)ethyl}aminobutyl] adamantamide;
- la N[4-{2-(2-méthylamino carbonyle phénoxy)ethyl]amino}butyl]benzamide ;
- la N[4-{2-(2-hydroxyphénoxy)ethyl}aminobutyl]benzamide ;
- la N[4-{2-(2-méthoxyphénoxy)ethyl}aminobutyl]cyclohexylamide ;
- la N[4-{2-(2-méthoxyphénoxy)ethyl}aminobutyl]cycloheptylamide ;
- la N[4-{2-(2-méthoxyphénoxy)ethyl}aminobutyl]2-bicyclo[2,2,1]heptylamide
ainsi que les sels de ces composés avec les acides minéraux ou organiques.

L'invention a également pour objet un procédé de préparation des produits de formule générale I telle que définie ci-dessus, caractérisé en ce que
**A)** soit l'on fait agir un produit de formule II 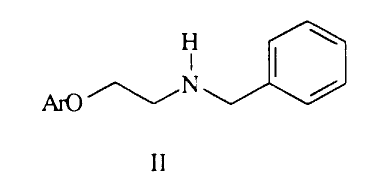 dans laquelle Ar a la signification indiquée ci-dessus, avec un produit de formule III 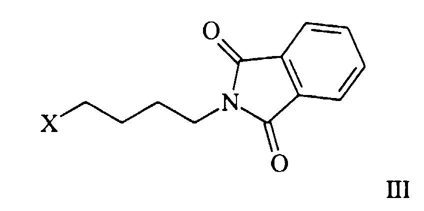 dans laquelle X représente un halogène ou un pseudo halogène pour obtenir un produit de formule IV 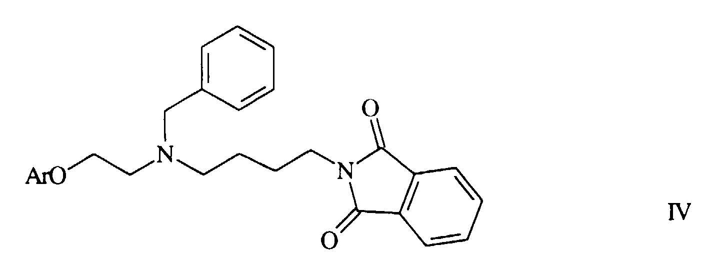 produit de formule IV que l'on soumet à une réaction d'élimination du groupement phtalimido pour obtenir un produit de formule V 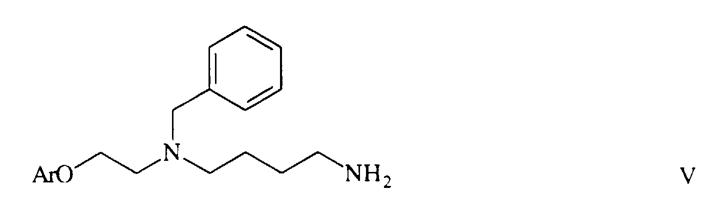 produit de formule V que l'on traite par réactif d'acylation dérivé de l'acide RCO₂H dans lequel R a la signification indiquée ci-dessus, pour obtenir un produit de formule VI 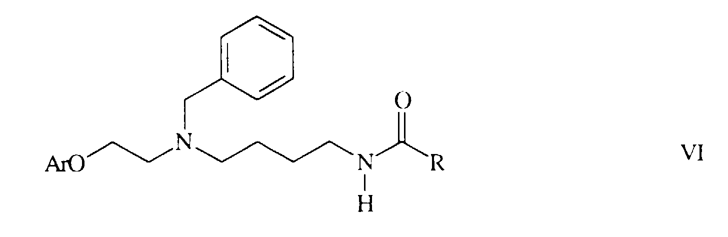
**B)** soit l'on fait agir un produit de formule VII 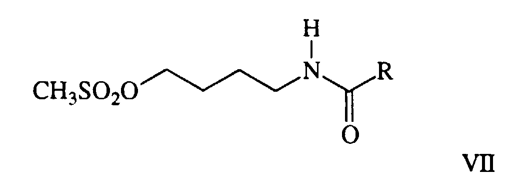 avec la N-benzyléthanolamine de formule 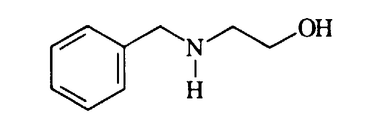 pour obtenir un produit de formule VIII 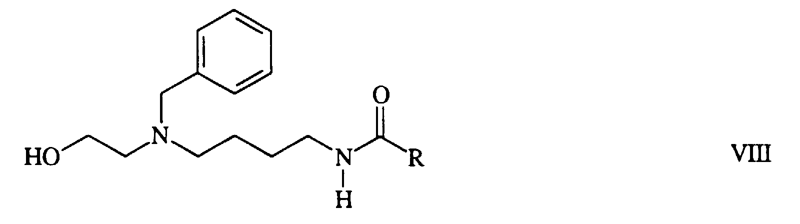 que l'on transforme en produit de formule IX 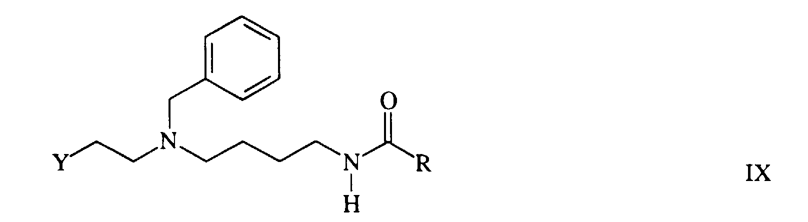 dans laquelle Y représente un radical halogène ou pseudo halogène, produit de formule IX que l'on transforme en produit de formule VI telle que définie précédemment en faisant agir un dérivé de phénol de formule ArOH, et produits de formule VI que l'on transforme en produit de formule I en clivant la fonction benzyle et produits de formule I que l'on transforme si désiré en sels d'acide par action de l'acide correspondant.

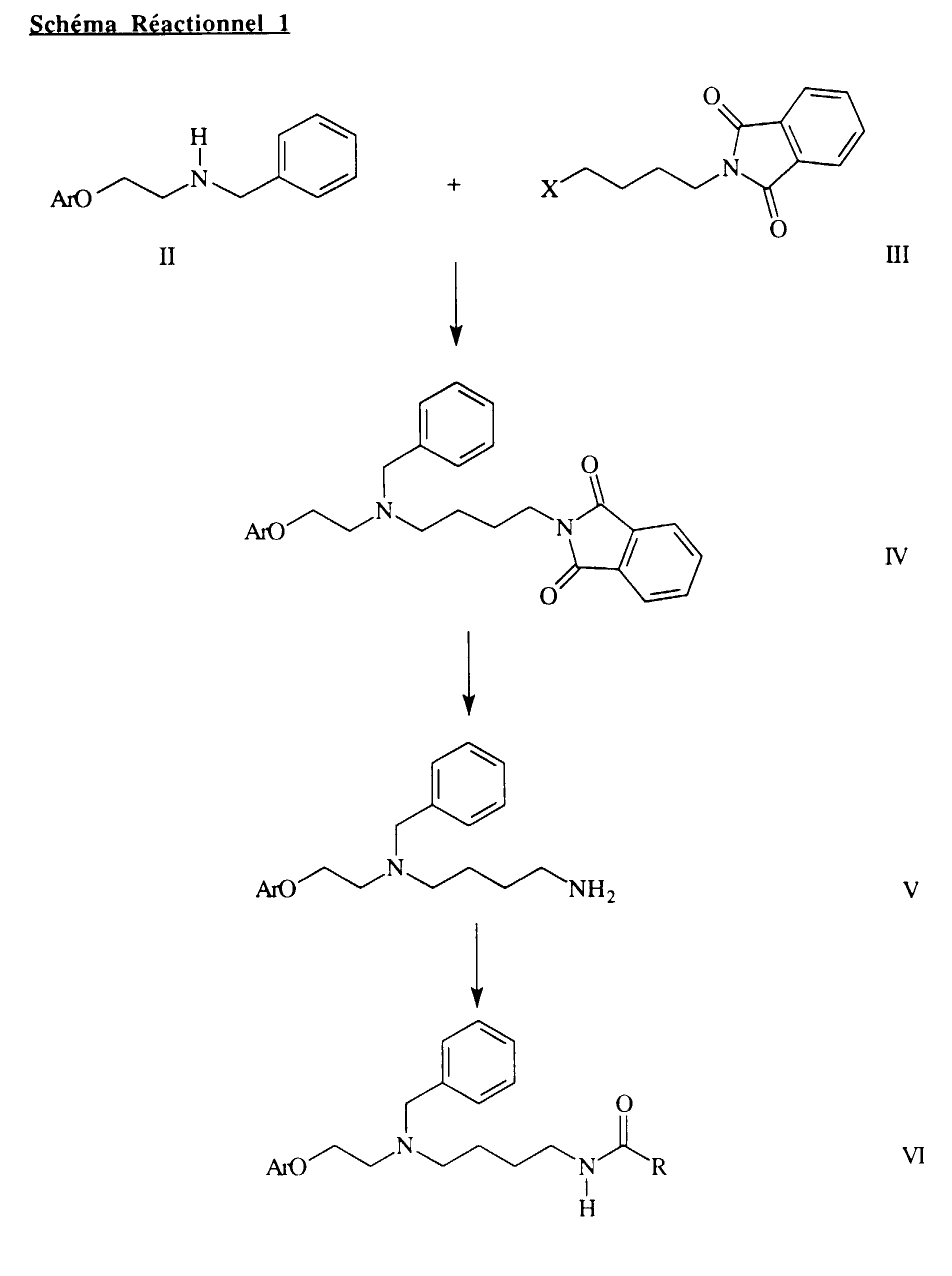

Dans ce schéma réactionnel, Ar et R sont tels que définis ci-dessus et X représente un groupe partant tel que chloro, bromo, iodo, méthanesulphonyloxy, benzènesulphonyloxy ou p-toluènesulphonyloxy en d'autres termes, un groupement halogène ou pseudo halogène.

La réaction d'un composé de formule générale II avec un composé de formule générale III pour obtenir un composé de formule générale IV peut être facilement réalisée en chauffant dans un solvant polaire, par exemple de l'acétonitrile ou du diméthylformamide, en présence d'un accepteur d'acide tel que le carbonate de potassium ou le carbonate de sodium. Les phthalimides de formule générale IV peuvent être transformés en amines primaires de formule générale V selon les méthodes conventionnelles, par exemple en chauffant avec de l'hydrate d'hydrazine, de préférence dans un solvant tel que l'éthanol. Les amines primaires de formule générale V ainsi obtenues peuvent être transformées en amides de formule générale VI par réaction avec un dérivé de l'acide RCO₂H qui peut être le chlorure d'acide ou l'anhydride d'acide approprié ou d'autres dérivés d'acides activés, tels que ceux utilisés habituellement dans les réactions de couplages de peptides. La réaction est réalisée dans un solvant inerte tel que l'éther, le tétrahydrofuranne ou le dichlorométhane et généralement en présence d'un accepteur d'acide tel qu'une amine tertiaire, par exemple, la triéthylamine ou la diisopropyléthylamine.

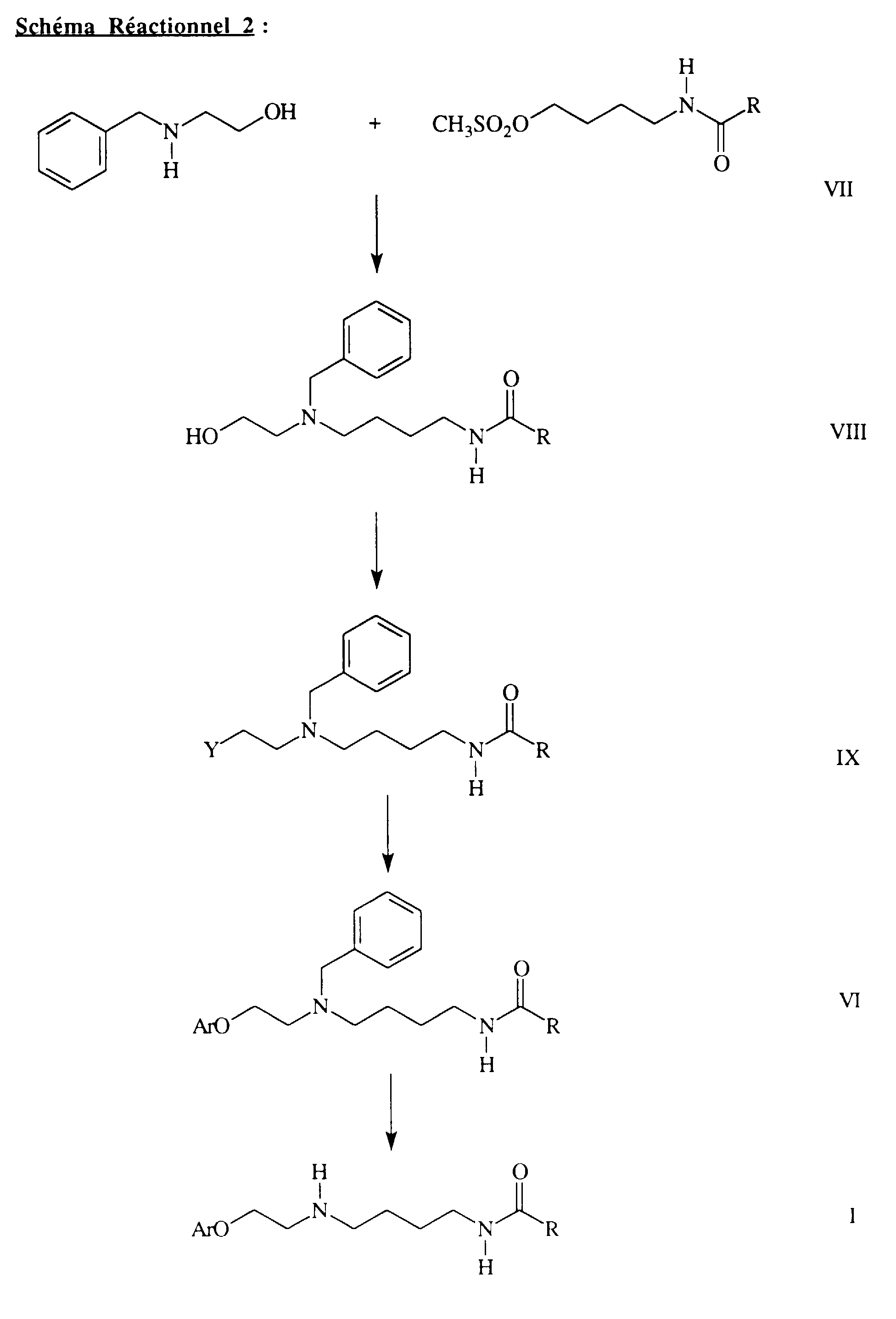

Dans ce schéma réactionnel, Ar et R sont tels que définis ci-dessus et Y, identique ou différent de X, représente un groupe partant tel que chloro, bromo, iodo, méthanesulphonyloxy, benzènesulphonyloxy, ou p-toluènesulphonyloxy, en d'autres termes un groupement halogène ou pseudo halogène.

Des composés de formule générale VIII peuvent être préparés en chauffant un composé de formule générale VII avec de la N-benzyléthanolamine dans un solvant polaire, tel qu'un alcool, en présence d'un accepteur d'acide tel qu'une amine tertiaire ou une base inorganique tel que le carbonate de sodium ou le carbonate de potassium. Alternativement, des composés de formule générale VIII peuvent être aisément préparés en chauffant simplement un composé de formule générale VII avec un excès de N-benzyléthanolamine en l'absence d'un solvant, de préférence sous atmosphère d'azote et à une température comprise entre 60° C et 90° C. Les composés de formule générale VIII ainsi obtenus peuvent être transformés, par exemple, en chlorures de formule générale IX (Y = Cl), par réaction avec du chlorure de méthanesulphonyl dans un solvant inerte, tel que le dichlorométhane, en présence d'une base organique telle que la triéthylamine ou la diisopropyléthylamine. Des composés de formule générale VI peuvent être préparés à partir de composés de formule générale IX en faisant réagir ces derniers avec un anion phénoxide produit à partir du phénol ArOH approprié, en utilisant une base tel que l'hydroxyde de sodium, l'hydroxyde de potassium ou l'hydrure de sodium. La réaction est réalisée dans un solvant aprotique et, de préférence, dans un solvant aprotique dipolaire, par exemple du diméthylformamide.

Les composés de formule générale I sont obtenus en déprotégeant les composés de formule générale VI selon les méthodes générales connues pour la débenzylation, par exemple, l'hydrogénation catalytique ou la réaction avec un chloroformate tel que le vinylchloroformate ou l'a-chloroéthylchloroformate suivie d'une hydrolyse ou d'une méthanolyse. D'autres méthodes de débenzylation, telles que celles décrites dans "Protective Groups in Organic Synthesis" [T.W. Green, P.G.M. Wuts ; 2nd Edition, J. Wiley and sons Inc., p. 364-6 (1991)] peuvent également être utilisées dans la mesure où elles sont compatibles avec les substituants avec le(s) noyau(x) aryle(s) des composés de formule générale VI.

La salification éventuelle des produits de formule I est également réalisée selon les méthodes usuelles indiquées ci-après dans la partie expérimentale.

Les composés de la présente invention possèdent d'intéressantes propriétés pharmacologiques. C'est ainsi que l'on a découvert que les composés de la présente invention ont une haute affinité pour le récepteur 5HT_{1A}.

Les composés de la présente invention peuvent ainsi être utilisés dans différentes applications thérapeutiques.

Les composés peuvent inhiber la sécrétion d'acide gastrique et le vomissement induit, par exemple, par le cis-platine. Ainsi, les composés de l'invention peuvent être utilisés en tant qu'anti-émétiques ou pour le traitement des maladies dans lesquelles il est nécessaire, ou souhaitable, de réduire la sécrétion gastrique acide, par exemple, les ulcères gastriques ou duodénaux, les gastrites, le reflux gastroesophagial, la dyspepsie gastrique, le syndrome de Zollinger-Ellison, les nausées.

Les composés de l'invention possèdent également une activité sur la vidange gastrique et la motilité intestinale. Par exemple, ils peuvent être utilisés pour lutter contre la constipation, l'atonie post-chirurgicale, la gastroparésie.

Ils peuvent également être utilisés pour lutter contre certaines maladies du système nerveux telles que l'anxiété, la dépression, les troubles du sommeil tels que l'insomnie, la dépendance vis à vis de certaines drogues, la maladie d'Alzheimer, les maladies de l'alimentation telles que l'anorexie, le vertige. Les composés de l'invention peuvent également être utilisés pour traiter des maladies du système cardiovasculaire, notamment l'hypertension.

On trouvera ci-après, dans la partie expérimentale, une illustration des propriétés pharmacologiques des composés de l'invention.

Ces propriétés rendent les produits de formule I aptes à une utilisation pharmaceutique. La présente demande a également pour objet, à titre de médicaments, les produits de formule I telle que définie ci-dessus, ainsi que les sels d'addition avec les acides minéraux ou organiques pharmaceutiquement acceptables desdits produits de formule I, ainsi que les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrée par intraveineuse.

L'invention a également pour objet l'utilisation des produits de formule I telle que définie ci-dessus, pour la préparation de médicaments antiémétiques, de médicaments destinés à réduire la sécrétion gastrique, de médicaments destinés à accélérer la vidange gastrique, de médicaments destinés à accélérer le transit intestinal, de médicaments destinés à traiter l'anxiété, la dépression, les troubles du sommeil ainsi que de médicaments destinés à traiter les maladies cardiovasculaires.

L'invention a également pour objet, à titre de produits industriels nouveaux, et notamment à titre de produits industriels nouveaux destinés à la préparation des produits de formule I, les produits de formules IV, V, VI, VIII et IX telles que décrites ci-dessus.

Les produits de départ de l'invention, en particulier les produits de formules II, III et VII, sont des produits connus ou qui peuvent être préparés à partir de produits connus. On peut citer les références suivantes : la N-benzyléthanolamine est un produit commercialisé par exemple par la firme ACROS.

Les produits de formule II peuvent être préparés par les méthodes classiques à partir des phénoxy éthylamines correspondantes, par exemple par l'intermédiaire de la benzamide suivie d'une réduction par l'hydrure de lithium aluminium ou une méthode équivalente. Alternativement, on peut utiliser une amination réductrice selon les méthodes usuelles.

Les phénoxyéthyléthylamines peuvent être préparées selon les méthodes usuelles, par exemple par réaction d'un phénol avec le chloracétonitrile en milieu basique, réaction suivie par la réduction du nitrile par l'hydrure de lithium aluminium selon la méthode décrite dans Chim. Ther. 8(3), 259-270 (1973).

Les produits de formule III sont commerciaux ou peuvent être fabriqués par les méthodes connues de l'homme de métier.

C'est ainsi que le produit de formule III, dans laquelle X représente un atome de brome, est commercialisé par la firme ACROS.

Les produits de formule III dans laquelle X représente un autre atome d'halogène tel que le chlore ou l'iode ou un pseudo halogène tel que les radicaux mésyle, tosyle, phénylsulfonyle peuvent être préparés par les méthodes usuelles à partir de l'alcool correspondant commercialisé par la firme MAYBRIDGE.

Les produits de formule VII peuvent être préparés à partir de la ω-hydroxybutyl amide, selon les méthodes connues pour la formation d'un mésylate à partir d'un alcool, par exemple selon la méthode décrite dans J. Org. Chem. 35(9), 3195-6 (1970).

Les exemples suivants sont présentés pour illustrer les procédures ci-dessus et ne doivent en aucun cas être considérés comme une limite à la portée de l'invention.

### PARTIE EXPÉRIMENTALE:

### Exemple 1

### N-[4-{2-(2-méthoxyphénoxy)éthyl}aminobutyl]benzamide (I, Ar = 2-méthoxyphényl, R = phényl : composé No. 1, Tableau 1)

### 1ère étape

### N-[4-{benzyl[2-(2-méthoxyphénoxy)éthyl]amino}butyl]phthalimide (IV, Ar = 2-méthoxyphényl)

On ajoute une solution de N-(4-bromobutyl)phthalimide (9,87 g, 0,035 mole) dans de l'acétonitrile (200 ml) à une solution chaude (60°C) de N-benzyl[2-(2-méthoxyphénoxy]éthylamine (8,5 g, 0,033 mole) dans de l'acétonitrile (170 ml) contenant du carbonate de potassium (9,1 g, 0,066 mole). Le mélange est agité et chauffé à reflux pendant 6 heures, puis laissé à refroidir jusqu'à la température ambiante, filtré et le solvant est évaporé sous pression réduite. Le résidu est repris dans du dichlorométhane (500 ml), lavé à l'eau (3 x 50 ml) et séché sur du sulfate de magnésium. La filtration et l'évaporation du solvant donnent une huile qui est purifiée par chromatographie flash sur du gel de silice en utilisant de l'acétate d'éthyle / heptane (1/4 puis 3/7) comme éluent afin d'obtenir 7,2 g (48 %) du composé IV sous forme d'huile. RMN (CDCl₃), δ : 1,5-1,7 (m, 4H), 2,54-2,67 (t, 2H), 2,84-2,97 (t, 2H), 3,59-3,73 (m, 4H), 3,83 (s, 3H), 3,99-4,15 (m, 2H), 6,79-6,89 (m, 4H), 7,20-7,32 (m, 5H), 7,64-7,88 (m, 4H).

### 2ème étape

### N-Benzyl,N-[2-(2-méthoxyphény)éthyl]-1,4-butanediamine (V, Ar = 2-méthoxyphényl)

On ajoute une solution d'hydrate d'hydrazine (0.83 ml, 0,017 mole) dans de l'éthanol (20 ml) à une solution de N-[4-{benzyl[2-(2-méthoxyphénoxy)éthyl]amino}butyl] phthalimide (7,2 g, 0,016 mole) dans de l'éthanol (70 ml) et la solution agitée est chauffée à reflux pendant 3 heures. Après refroidissement à température ambiante, le solvant est évaporé sous pression réduite et l'huile résiduelle est traitée avec de l'acide chlorhydrique 1N (40 ml). Le précipité formé est éliminé par filtration, lavé à l'eau et le filtrat et les liquides de lavage sont basifiés avec du carbonate de potassium (16 g). L'huile séparée est extraite avec du dichlorométhane (3 x 80 ml) et les extraits séchés sur du sulfate de magnésium. La filtration et l'évaporation du solvant sous pression réduite donnent 4,4 g (86 %) du composé attendu sous forme d'huile. Le traitement d'une solution éthérée de la base-libre avec une solution saturée de gaz de chlorure d'hydrogène dans de l'éther, permet d'obtenir la N-benzyl-N-[2-(2-méthoxyphénoxy) éthyl]-1,4-butanediamine comme sel dihydrochloré ; pf 200-202° C après recristallisation à partir de l'éthanol.

### 3ème étape

### N-[4-{benzyl[2-(2-méthoxyphénoxy)éthyl]amino}butyl]benzamide (VI, Ar = 2-méthoxyphényle, R = phényle)

On ajoute, sous agitation sous refroidissement (5-10° C), une solution de chlorure de benzoyle (0,9 ml, 0,0076 mole) dans du tétrahydrofuranne (25 ml) à une solution de triéthylamine (1,05 ml, 0,0076 mole) dans du tétrahydrofuranne (25 ml). Après 30 minutes à 10° C, puis 1,5 heures à température ambiante, le solvant est évaporé sous pression réduite. Le résidu est repris dans du dichlorométhane, la solution est lavée à l'eau et séchée sur du sulfate de magnésium. La filtration et l'évaporation du solvant sous pression réduite donnent une huile qui est purifiée par chromatographie flash sur gel de silice, en utilisant de l'acétate d'éthyle / hexane (1/1 puis 3/2) comme éluant pour obtenir 2 g (61 %) du composé attendu sous forme d'huile.
RMN (CDCl₃), δ : 1,58-1,85 (m, 4H), 2,60-2,66 (m, 2H), 2,85-2,97 (t, 2H), 3,39-3,45 (m, 2H), 3,66 (s, 2H), 3,8 (s, 3H), 3,99-4,12 (t, 2H), 6,42 (large s, 1H), 6,76-6,90 (m, 4H), 7,21-7,46 (m, 8H), 7,66-7,77 (m, 2H).

### 4ème étape

### N-[4-{2-(2-méthoxyphénoxy)éthyl}aminobutyl]benzamide (I, Ar = 2-méthoxyphényle, R = phényle : composé No. 1, Tableau 1)

On ajoute un catalyseur constitué par du palladium sur carbone (0,8 g de 10 %) à une solution de N-[4-{henzyl[2-(2-méthoxyphénoxy)éthyl]amino}butyl]benzamide (2 g, 0,0046 mole) dans du méthanol (30 ml) et le mélange est hydrogéné à 1,5 psi à température ambiante. Lorsque l'absorption d'hydrogène est terminée (environ 3 heures), le catalyseur est éliminé par filtration et le solvant est évaporé sous pression réduite pour obtenir 1,2 g (76 %) du composé I sous forme d'huile. Le traitement d'une solution éthérée de la base-libre avec une solution saturée d'acide chlorhydrique gazeux dans de l'éther donne 1,0 g (59 %) de dichlorhydrate du composé No. 1 sous forme de cristaux blancs, pf 118-120° C.

### Exemple 2

### N-[4-{2-(2-méthoxyphénoxy)éthyl}aminobutyl]-1-adamantamide (I, Ar = 2-méthoxyphényle, R = 1-adamantyle : composé No. 2, Tableau 1)

### 1ère étape

### N-[4-{benzyl[2-(2-méthoxyphénoxy)éthyl]amino}butyl]-1-adamantamide (VI, Ar = 2-méthoxyphényle, R = 1-adamantyle)

On ajoute du chlorure de 1-adamantylcarbonyle (2,14 g, 0,011 mole), par portions, dans une solution refroidie (5° C) de N-benzyl-N-[2-(2-méthoxyphénoxy)éthyl]1,4-butanediamine (3,28 g, 0,01 mole), préparée selon la méthode décrite dans l'exemple 1, et de la triéthylamine (1,54 ml, 0,011 mole) dans du tétrahydrofuranne (40 ml). Après 30 minutes, le mélange réactionnel est réchauffé à température ambiante et, une heure plus tard, le solvant est évaporé sous pression réduite. Le résidu est repris dans du dichlorométhane, lavé à l'eau et séché sur du sulfate de magnésium. La filtration et l'évaporation du solvant sous pression réduite donnent une huile qui est purifiée par chromatographie flash sur gel de silice, en utilisant de l'acétate d'éthyle / hexane (1/2 puis 3/4) comme éluant pour obtenir 3 g (61 %) du composé attendu sous forme d'huile.
RMN (CDCl₃), δ : 1,24-1,29 (m, 2H), 1,53-1,67 (m, 4H), 1,67-1,94 (m, 14H), 2,5-2,7 (m, 2H), 2,93-2,96 (t, 2H), 3,21-3,23 (m, 2H), 3,71 (s, 2H), 3,85 (s, 3H), 4,07-4,10 (t, 2H), 6,82-6,92 (m, 4H), 7,24-7,37 (m, 5H).

### 2ème étape

### N-[4-{2-(2-méthoxyphénoxy)éthyl}aminobutyl]-1-adamantamide (I, Ar = 2-méthoxyphénoxy, R = 1-adamantyl : composé No. 2, Tableau 1)

On ajoute 1,5 g d'un catalyseur constitué par du palladium sur carbone à 10 % à une solution de N-[4-{benzyl[2-(2-méthoxyphénoxy)éthyl]amino}butyl]-1-adamantamide (2,85 g, 0,058 mole) dans du méthanol (30 ml) et le mélange est hydrogéné à 1,5 psi à température ambiante. Lorsque l'absorption d'hydrogène est terminée (environ 3 heures), le catalyseur est éliminé par filtration et le solvant évaporé sous pression réduite, pour donner 2 g (87 %) du composé I sous forme d'huile. Le traitement d'une solution de cette base-libre (0,6 g) dans de l'acétone avec une solution d'acide fumarique (0,17 g) dans de l'acétone produit 0.5 g du fumarate du composé No. 2 sous forme de cristaux blancs, pf 122-124° C.

### Exemple 3

### N-(4-{2-(2-nitrophénoxy)éthyl]amino}butyl]benzamide (I, Ar = 2-nitrophényle, R = phényle : composé No. 8, Tableau 1)

### 1ère étape

### N-[4-{benzyl(2-hydroxyéthyl)amino}butyl]benzamide (VIII, R = phényle)

On chauffe un mélange de N-benzylaminoéthanol (60,5 g, 56,8 ml, 0,4 mole) et de N-[4-(méthanesulphonyloxy)butyl]benzamide (51,5 g, 0,19 mole) pendant 2 heures à 75-80° C, sous atmosphère d'azote. Le mélange réactionnel est refroidi, repris dans du dichlorométhane (400 ml), la solution est lavée à l'eau (3 x 100 ml), puis séchée sur du sulfate de magnésium. La solution est filtrée et le solvant éliminé sous pression réduite pour obtenir une huile qui cristallise avec le diisopropyle éther, afin d'obtenir 51,5 g (83 %) du composé VIII sous forme de cristaux blancs, pf 61-62° C.

### 2ème étape

### N-[4-{benzyl(2-chloroéthyl)amino}butyl]benzamide (IX, R = phényle, Y = Cl)

On ajoute goutte à goutte du chlorure de méthanesulphonyle (14,8 g, 10 ml, 0,13 mole), sous agitation, à une solution refroidie (5° C) de N-[4{benzyl(2-hydroxyéthyl)amino}butyl] benzamide (35 g, 0,11 mole) dans du dichlorométhane (300 ml). La solution est ramenée à température ambiante et, après 1,5 heure, on ajoute de la triéthylamine (13 g, 18 ml, 0,13 mole) et on maintient l'agitation pendant encore 1,5 heure. Le mélange réactionnel est lavé à l'eau glacée (150 ml), à l'eau salée saturée (2 x 100 ml) et séchée sur du sulfate de magnésium. La filtration et l'évaporation du solvant sous pression réduite donnent 36,6 g (99 %) du composé attendu sous forme d'huile marron clair, qui cristallise en se maintenant à 4° C. RMN (CDCl₃), δ : 1,58-1,67 (m, 4H), 2,58 (m, 2H), 2,83 (t, 2H, J = 6,8 Hz), 3,40-3,45 (q, 2H, J = 6,5 Hz), 3,51 (t, 2H, J = 6,8 Hz), 3,66 (s, 2H), 6,19 (s. 1H), 7,24-7,48 (m, 8H), 7,73-7,75 (m, 2H).

### 3ème étape

### N-[4-{henzyl[2-(2-nitrophénoxy)éthyl]amino}butyl]benzamide (VI, Ar = 2-nitrophényle, R = phényle)

On ajoute du 2-nitrophénol (0,56 g, 0,004 mole) par portions à une suspension agitée d'hydrure de sodium (160 mg d'une dispersion à 60 % dans de l'huile, 0,004 mole) dans du diméthylformamide sec (10 ml), sous atmosphère d'azote. Lorsque l'évolution de l'hydrogène est terminée, on ajoute goutte à goutte, sous agitation, une solution de N-[4-{benzyl(2-chloroéthyl)amino}butyl]benzamide (1,6 g, 0,0046 mole) dans du diméthylformamide sec (5 ml). Le mélange réactionnel est chauffé à 65-70° C pendant 1,5 heure, refroidi à température ambiante et versé sur de la glace pilée. Le mélange est extrait avec de l'éther (50 ml), la phase organique est séparée et lavée à l'eau (3 x 20 ml) avant d'être séchée sur du sulfate de magnésium. La filtration et l'évaporation du solvant sous pression réduite donnent 1,3 g d'une huile qui est purifiée par chromatographie flash sur gel de silice, en utilisant un mélange d'acétate d'éthyle / heptane (3/1) comme éluant. Le composé attendu est obtenu sous forme d'huile (0,94 g, 52 %).
RMN (CDCl₃), δ : 1,59-1,65 (m, 4H), 2,62 (t, 2H, J = 0,65 Hz), 2,92 (t, 2H, J = 0,55 Hz), 3,42-3,45 (m, 2H), 3,67 (s, 2H), 4,09 (t, 2H, J = 0,55 Hz), 6,45 (s, 1H), 6,95-6,98 (m, 2H), 7,20-7,32 (m, 5H), 7,38-7,46 (m, 4H), 7,76-7,79 (m, 3H).

### 4ème étape

### N-[4-{2-(2-nitrophénoxy)éthyl)amino}butyl]benzamide (I, Ar = 2-nitrophényle, R = phényle : composé No. 8, Tableau 1)

On ajoute goutte à goutte une solution de a-chloroéthylchloroformate (0,28 g, 0,002 mole) dans du dichloroéthane sec (1,8 ml) à une solution de N-[4-{benzyl[2-(2-nitroxyphénoxy)éthyl]amino}butyl]benzamide (0,82 g, 0,18 mole) dans du dichloroéthane sec (9 ml), sous atmosphère d'azote et avec un refroidissement dans un bain de glace. Au bout de 2 heures, le solvant est évaporé sous pression réduite, le résidu est repris dans du méthanol (10 ml) et chauffé pendant 1 heure à reflux. Le solvant est concentré sous pression réduite et on ajoute de l'acétate éthyle (10 ml). Le précipité formé est recueilli par filtration, lavé avec de l'acétate éthyle, du diéthyl éther et séché pour obtenir 0,55 g (76 %) du composé I sous forme de chlorhydrate, pf 159,5-160° C.

### Exemple 4

### N-[4-{2-(2-méthylaminocarbonylphénoxy)éthyl]amino}butyl]benzamide (I, Ar = 2-MeNHC(O)C₆H₄, R = phényl : composé No. 18, Tahleau 1)

### 1ère étape

### N-[4-{benzyl[2-(2-méthylaminocarbonylphénoxy)éthyl]amino}butyl]benzamide (VI, Ar = 2-MeNHC(O)C₆H₄, R = phényle)

On ajoute une solution de N-[4-{benzyl(2-chloroéthyl)amino}butyl]benzamide (8,1 g, 0,023 mole), préparé selon la méthode décrite dans l'exemple 3, dans du diméthylformamide (50 ml) à une solution agitée de 2-méthylaminocarbonylphénoxide de sodium, préparée *in situ* à partir de 2-méthylaminocarbonylphénol (3,0 g, 0,02 mole), d'hydroxyde de sodium (0,8 g, 0,02 mole) et d'eau (4 ml) dans du diméthylformamide (50 ml). Le mélange est chauffé à 85° C pendant 4 heures, refroidi à température ambiante et versé dans un mélange de glace et d'eau. Le mélange est extrait avec de l'acétate éthyle (250 ml) et la phase organique est lavée avec 2 % d'hydroxyde de sodium aqueux (100 ml), de l'eau salée (3 x 50 ml) et séchée sur du sulfate de magnésium. La filtration et l'évaporation du solvant sous pression réduite donnent une huile jaune qui est purifiée par chromatographie flash sur gel de silice en utilisant de l'acétate éthyle comme éluant, afin d'obtenir 5,2 g (57 %) du composé VI sous forme d'huile.
RMN (CDCl₃), δ : 1,35-1,80 (m, 4H), 2,40-2,65 (m, 2H), 2,84-2,94 (m, 4H), 3,34-3,44 (m, 2H), 3,65 (s, 2H), 4,16 (t, 2H, J = 5,5 Hz), 6,30-6,65 (m, 1H), 6,84-7,09 (m, 2H), 7,26-7,48 (m, 8H), 7,72-7,82 (m, 2H), 8,13-8,23 (m, 2H).

### 2ème étape

### N-[4-{2-(2-méthylaminocarbonylphénoxy)éthyl]amino}butyl]benzamide (I, Ar = 2-MeNHC(O)C₆H₄, R = phényl : composé No. 18, Tableau 1)

On ajoute un catalyseur constitué par du palladium sur carbone à 10 %, desactivé à l'eau, à une solution de N-[4-{benzyl[2-(2-méthylaminocarbonylphénoxy) éthyl] amino} butyl] benzamide (6,6 g, 0,015 mole) dans du méthanol (200 ml) et le mélange est hydrogéné sous pression atmosphérique et à température ambiante. Lorsque l'absorption de l'hydrogène est terminée (environ 3 heures), le catalyseur est éliminé par filtration et le filtrat évaporé sous pression réduite. Le résidu est recueilli dans de l'acétone (15 ml) et traité avec une solution d'acide fumarique (1,04 g, 0,009 mole) dans de l'acétone (100 ml). Le solide huileux qui se sépare par précipitation, cristallise avec l'ajout d'isopropanol (50 ml). Le produit cristallin blanc est recueilli par filtration, lavé avec de l'acétone et séché pour obtenir 2,7 g (38 %) du composé No. 18 sous forme d'hémifumarate, pf 159-161° C.

Les procédés décrits ci-dessus donnent un composé de l'invention sous forme d'une base-libre ou d'un sel d'addition avec un acide. Si le composé de l'invention est obtenu sous la forme d'un sel d'addition avec un acide, la base libre peut être obtenue en basifiant une solution du sel d'addition avec un acide. A l'inverse, si le produit du procédé est une base libre, le sel d'addition avec un acide, en particulier un sel d'addition avec un acide pharmaceutiquement acceptable, peut être obtenu en dissolvant la base libre dans un solvant organique approprié et en traitant la solution avec un acide, selon les procédures conventionnelles de préparation des sels d'addition avec un acide à partir des bases libres.

Des exemples de sels d'addition avec un acide sont ceux dérivés d'acides inorganiques tels que les acides sulfurique, chlorhydrique, bromhydrique ou phosphorique, ou d'acides organiques tels que les acides tartrique, fumarique, maléique, citrique, caprylique, oxalique, benzoïque, méthanesulphonique, p-toluènesulphonique, benzènesulphonique, succinique ou acétique.

Pour les composés de l'invention contenant un centre asymétrique, les mélanges racémiques et les isomères optiquement actifs individuels sont également considérés comme faisant partie de la portée de l'invention.

Le Tableau 1 ci-dessous montre les principaux composés préparés selon les procédures ci-dessus et qui illustrent l'invention sans en limiter la portée. Les composés Nos. 1, 2, 8 et 18 correspondent aux produits des exemples 1, 2, 3 et 4 décrits ci-dessus. Les autres produits ont été préparés en utilisant le même procédé.

**Tableau 1**

| **Composé No.** | **Ar** | **R** | **Sel** | **pf (°C)** |
|---|---|---|---|---|
| 1 | 2-MeO.C₆H₄ | C₆H₅ | chlorhydrate | 118-120 |
| 2 | 2-MeO.C₆H₄ | 1-adamantyle | fumarate | 122-124 |
| 3 | 2-MeO.C₆H₄ | 2-pyridyle | dichlorhydrate | 152-154 |
| 4 | 2-MeO.C₆H₄ | 2-isoquinolyle | dichlorhydrate | 166-168 |
| 5 | 2-ⁱPrO.C₆H₄ | C₆H₅ | chlorhydrate | 108-110 |
| 6 | 2-H₂NC(O).C₆H₄ | C₆H₅ | chlorhydrate | 128-132 |
| 7 | 2-MeO.C₆H₄ | 2-MeC(O)NH.C₆H₄ | chlorhydrate | 92-94 |
| 8 | 2-O₂N.C₆H₄ | C₆H₅ | chlorhydrate | 159,5-160 |
| 9 | 2-NC.C₆H₄ | C₆H₅ | chlorhydrate | 168-168,5 |
| 10 | 2-HOCH₂.C₆H₄ | C₆H₅ | chlorhydrate | 144-145,5 |
| 11 | 2-MeS.C₆H₄ | C₆H₅ | chlorhydrate | 140-140,5 |
| 12 | 2-MeO.C₆H₄ | 2-HO.C₆H₄ | hémifumarate | 140-142 |
| 13 | 2-MeO.C₆H₄ | 2-MeO.C₆H₄ | fumarate | 120-122 |
| 14 | 2-MeO.C₆H₄ | 3-MeO.C₆H₄ | fumarate | 68-70 |
| 15 | 2-MeO.C₆H₄ | 4-MeCH(OH).C₆H₄ | hémifumarate | 152-154 |
| 16 | 2-MeC(O).C₆H₄ | C₆H₅ | chlrohydrate | 187-192,5 |
| 17 | 2-MeO.C₆H₄ | 4-MeO.C₆H₄ | hémifumarate | 135-137 |
| 18 | 2-MeNHC(O).C₆H₄ | C₆H₅ | hémifumarate | 159-161 |
| 19 | 3-H₂NC(O).C₆H₄ | C₆H₅ | chlorhydrate | 264-266,5 |
| 20 | 2-HO.C₆H₄ | C₆H₅ | chlorhydrate | 252-254,5 |
| 21 | 2-MeC(O)NH.C₆H₄ | C₆H₅ | base libre | 74-78 |
| 22 | 2-MeO.C₆H₄ | 4-O₂N.C₆H₄ | chlorhydrate | 162,5-163,5 |
| 23 | 2-MeO.C₆H₄ | 4-F.C₆H₄ | chlorhydrate | 119-120,5 |
| 24 | 2-F.C₆H₄ | C₆H₅ | chlorhydrate | 167-168 |
| 25 | 3-MeO.C₆H₄ | C₆H₅ | chlorhydrate | 174-175,5 |
| 26 | 2-MeO.C₆H₄ | ^{t}butyl | fumarate | 149-151 |
| 27 | 2-Me.C₆H₄ | C₆H₅ | chlorhydrate | 162-163 |
| 28 | 2,3-(MeO)₂.C₆H₃ | C₆H₅ | chlorhydrate | 141,5-142 |
| 29 | 2-MeO.C₆H₄ | 2,6-Me₂.C₆H₃ | fumarate | 123-125 |
| 30 | 2-MeO.C₆H₄ | 3-I.C₆H₄ | chlorhydrate | 121-121,5 |
| 31 | 3-MeNHC(O).C₆H₄ | C₆H₅ | chlorhydrate | 227-229 |
| 32 | 2-Me₂NC(O).C₆H₄ | C₆H₅ | fumarate | 126-128,5 |
| 33 | 2-MeO.C₆H₄ | CH₂^{t}Bu | fumarate | 130-132 |
| 34 | 2-MeO.C₆H₄ | cyclohexyle | fumarate | 134-136 |
| 35 | 2-MeO.C₆H₄ | isopropyle | fumarate | 120-122 |
| 36 | 2-HONH(CO).C₆H₄ | C₆H₅ | oxalate | 147-148,5 |
| 37 | 2,6-(MeO)₂.C₆H₃ | C₆H₅ | oxalate | 166-167,5 |
| 38 | 2-H₂NC(O)NH.C₆H₄ | C₆H₅ | chlorhydrate | 193-194 |
| 39 | 2-MeO.C₆H₄ | (CH₂)₄Me | fumarate | 110-112 |
| 40 | 2-MeNHC(O)NHCH₂.C₆H₄ | C₆H₅ | oxalate | 170-171,5 |
| 41 | 2-MeO.C₆H₄ | CH(ⁿPr)₂ | fumarate | 118-120 |
| 42 | 2-MeO.C₆H₄ | CH₂CH(Me)₂ | fumarate | 134-136 |
| 43 | 2-MeO.C₆H₄ | cyclopentyle | fumarate | 122-124 |
| 44 | 2-MeO₂C.C₆H₄ | C₆H₅ | chlorhydrate | 103-106 |
| 45 | 2-MeO.C₆H₄ | 1-méthylcyclohexyl | fumarate | 120-122 |
| 46 | 2-MeO.C₆H₄ | cycloheptyle | fumarate | 125-126 |
| 47 | 2-MeNHC(O).C₆H₄ | CH₂^{t}Bu | hémifumarate | 137-138 |
| 48 | 3-MeNHC(O).C₆H₄ | ^{t}Bu | chlorhydrate | 169-171,5 |
| 49 | 3-MeC(O)NH.C₆H₄ | ^{t}Bu | chlorhydrate | 164-165,5 |
| 50 | 2-MeO.C₆H₄ | 2-bicyclo[2,2,1]heptyl | fumarate | 130-131 |
| 51 | 2-MeNHC(O).C₆H₄ | ^{t}Bu | fumarate | 134-135,5 |
| 52 | 3-MeNHC(O).C₆H₄ | CH₂^{t}Bu | hémifumarate | 145-146 |
| 53 | 2-MeO.C₆H₄ | CH(Et)₂ | hémifumarate | 134-135 |
| 54 | 2-MeO.C₆H₄ | C(Et)Me₂ | fumarate (0,75) | 115-116 |
| 55 | 3-MeO.C₆H₄ | ^{t}Bu | fumarate | 148,5-149 |
| 56 | 2-MeO.C₆H₄ | 4-méthylcyclohexyl | fumarate (0,75) | 127-128 |
| 57 | 3-EtO₂CNH.C₆H₄ | C₆H₅ | oxalate | 175-177 |
| 58 | 2-MeNHC(O).C₆H₄ | 4-F.C₆H₄ | hémifumarate | 154,5-156 |
| 59 | 2-MeO.C₆H₄ | CH₂ cyclopentyle | fumarate | 118-119 |
| 60 | 3-H₂NC(O)NH.C₆H₄ | C₆H₅ | oxalate | 141-143 |
| 61 | 2-MeNHC(O).C₆H₄ | cyclohexyle | oxalate | 180-182 |
| 62 | 3-MeNHC(O).C₆H₄ | cyclohexyle | oxalate | 171-173 |
| 63 | 3-MeC(O)NH.C₆H₄ | CH₂^{t}Bu | hémifumarate | 166-167 |
| 64 | 2-EtO.C₆H₄ | ^{t}Bu | fumarate | 131,5-133 |
| 65 | 2-HONC(O).C₆H₄ | cyclohexyle | chlorhydrate | 179-180 |
| 66 | 3-EtO₂CNH.C₆H₄ | cyclohexyle | chlorhydrate | 204,5-205 |
| 67 | 3-MeO₂C.C₆H₄ | CH₂^{t}Bu | chlorhydrate | 157-158 |
| 68 | 3-H₂NC(O)NH.C₆H₄ | CH₂^{t}Bu | chlorhydrate | 144-146 |
| 69 | 2-MeO.C₆H₄ | cyclopropyl | fumarate | 90-92 |

En utilisant le procédé indiqué ci-dessus, on peut également préparer les produits suivants, qui font également partie de l'invention et qui constituent des produits préférés :

| **Composé** | **Ar** | **R** |
|---|---|---|
| A | 2-HONHC(O).C₆H₄ | 4-F.C₆H₄ |
| B | 2-HONHC(O).C₆H₄ | cycloheptyl |
| C | 2-HONHC(O).C₆H₄ | cyclopentyl |
| D | 2-HONHC(O).C₆H₄ | cyclopentylCH₂ |
| E | 2-HONHC(O).C₆H₄ | adamantyl |
| F | 2-HONHC(O).C₆H₄ | ^{t}butyl |
| G | 2-HONHC(O).C₆H₄ | ^{t}butylCH₂ |
| H | 2-MeNHC(O).C₆H₄ | cycloheptyl |
| I | 2-MeNHC(O).C₆H₄ | cyclopentyl |
| J | 2-MeNHC(O).C₆H₄ | cyclopentylCH₂ |
| K | 2-MeNHC(O).C₆H₄ | adamantyl |
| L | 2-HO.C₆H₄ | 4-F.C₆H₄ |
| M | 2-HO.C₆H₄ | cycloheptyl |
| N | 2-HO.C₆H₄ | cyclohexyl |
| O | 2-HO.C₆H₄ | cyclopentyl |
| P | 2-HO.C₆H₄ | cyclopentylCH₂ |
| Q | 2-HO.C₆H₄ | adamantyl |
| R | 2-HO.C₆H₄ | ^{t}butyl |
| S | 2-HO.C₆H₄ | ^{t}butylCH₂ |
| T | 3-H₂NC(O)NH.C₆H₄ | 4-F.C₆H₄ |
| U | 3-H₂NC(O)NH.C₆H₄ | cycloheptyl |
| V | 3-H₂NC(O)NH.C₆H₄ | cyclohexyl |
| W | 3-H₂NC(O)NH.C₆H₄ | cyclopentyl |
| X | 3-H₂NC(O)NH.C₆H₄ | cyclopentylCH₂ |
| Y | 3-H₂NC(O)NH.C₆H₄ | adamantyl |
| Z | 3-H₂NC(O)NH.C₆H₄ | ^{t}butyl |
| AA | 3-EtO₂CNH.C₆H₄ | 4-F.C₆H₄ |
| AB | 3-EtO₂CNH.C₆H₄ | cycloheptyl |
| AC | 3-EtO₂CNH.C₆H₄ | cyclopentyl |
| AD | 3-EtO₂CNH.C₆H₄ | cyclopentylCH₂ |
| AE | 3-EtO₂CNH.C₆H₄ | adamantyl |
| AF | 3-EtO₂CNH.C₆H₄ | ^{t}butyl |
| AG | 3-EtO₂CNH.C₆H₄ | ^{t}butylCH₂ |
| AH | 3-MeO₂CNH.C₆H₄ | 4-F.C₆H₄ |
| AI | 3-MeO₂CNH.C₆H₄ | C₆H₅ |
| AJ | 3-MeO₂CNH.C₆H₄ | cycloheptyl |
| AK | 3-MeO₂CNH.C₆H₄ | cyclohexyl |
| AL | 3-MeO₂CNH.C₆H₄ | cyclopentyl |
| AM | 3-MeO₂CNH.C₆H₄ | cyclopentylCH₂ |
| AN | 3-MeO₂CNH.C₆H₄ | adamantyl |
| AO | 3-MeO₂CNH.C₆H₄ | ^{t}butyl |
| AP | 3-MeC(O)NH.C₆H₄ | 4-F.C₆H₄ |
| AQ | 3-MeC(O)NH.C₆H₄ | C₆H₅ |
| AR | 3-MeC(O)NH.C₆H₄ | cycloheptyl |
| AS | 3-MeC(O)NH.C₆H₄ | cyclohexyl |
| AT | 3-MeC(O)NH.C₆H₄ | cyclopentyl |
| AU | 3-MeC(O)NH.C₆H₄ | cyclopentylCH₂ |
| AV | 3-MeC(O)NH.C₆H₄ | adamantyl |
| AW | 3-MeNHC(O).C₆H₄ | 4-F.C₆H₄ |
| AX | 3-MeNHC(O).C₆H₄ | cycloheptyl |
| AY | 3-MeNHC(O).C₆H₄ | cyclopentyl |
| AZ | 3-MeNHC(O).C₆H₄ | cyclopentylCH₂ |
| BA | 3-MeNHC(O).C₆H₄ | adamantyl |

### Etude pharmacologique des produits de l'invention :

### 1) Affinité des composés de l'invention pour le récepteur 5-HT_{1A} :

L'affinité des composés pour les récepteurs 5-HT_{1A} sérotonergiques est déterminée en mesurant l'inhibition de [3H]8-hydroxy-2(di-n-propylamino)tétralin([3H]8-OH-DPAT) lié au cortex cérébral du rat, selon la méthode de Peroutka et ses collaborateurs [(J. Neurochem., 47, 529 (1986)].

Des cortex cérébraux de rats Sprague Dawley mâles sont homogénéisés dans du Tris-HCl 50 mM, pH = 7,4 et centrifugés à 40 000 g pendant 10 min à 4° C. Des pastilles sont remises en suspension dans le même tampon et incubées pendant 10 min à 37° C, et les homogénats sont de nouveau centrifugés à 40 000 g pendant 10 min à 4° C.

Des essais d'inhibition compétitive de liaison de [3H]8-OH-DPAT sont réalisés trois fois avec des compétiteurs non marqués, avec des concentrations comprises entre 100 pM et 100 µM. Des membranes de cortex cérébraux de rats (10 mg en poids mouillé / ml) sont incubées avec du [3H]8-OH-DPAT (1 nM) pendant 30 min à 25° C dans du Tris-HCl 50 mM, pH = 7,4 contenant 4 mM de CaCl₂, 10 µM de pargyline et 0,1 % d'acide ascorbique.

Le [3H]8-OH-DPAT lié est séparé du [3H]8-OH-DPAT libre par filtration immédiate au moyen de filtres en fibre de verre Whatman GF/B, en utilisant un récupérateur de cellules Brandel. Les filtres sont lavés trois fois avec le même tampon à 0-4° C et leur radioactivité est étudiée au moyen d'un spectromètre à scintillation liquide.

La liaison spécifique est obtenue en soustrayant la liaison déterminée en présence de 1 µM de 8-OH-DPAT de la liaison totale. Les caractéristiques de la liaison sont analysées par analyse itérative de la régression non linéaire par ordinateur, en utilisant le programme Ligand [Munson and Rodbard, Anal. Biochem., 107, 220 (1980)].

Les résultats pour les composés représentatifs de l'invention sont donnés dans le Tableau 2. Le Buspirone, l'agoniste 5-HT_{1A} utilisé cliniquement, est cité en tant que référence.

**Tableau 2**

| **Composé No.** | **Ki(nM)** |
|---|---|
| 1 | 0,20 |
| 2 | 0,10 |
| 3 | 1,4 |
| il | 0,71 |
| 14 | 1,6 |
| 20 | 0,23 |
| 23 | 0,33 |
| 30 | 0,44 |
| 33 | 0,33 |
| 34 | 0,087 |
| 45 | 0,14 |
| 46 | 0,094 |
| 50 | 0,071 |
| 59 | 0,65 |
| 64 | 0,77 |
| Buspirone | 20 |

### 2) Activité anti-sécrétoire gastrique :

L'activité anti-sécrétoire gastrique des composés de l'invention est testée selon la méthode décrite par Shay et ses collaborateurs [H. Shay, D.C. Sun, M. Gruenstein, **Gastroenterology**, 26, 906 (1954)]. Ainsi, des rats Sprague Dawley mâles pesant environ 200 g, avec accès libre à de l'eau, sont mis à jeun 24 heures avant les expérimentations. On ligature les pylores sous anesthésie à l'éther et les médicaments à tester sont simultanément administrés soit par voie intra-péritonéale (i.p.) ou par voie intra-duodénale (i.d.). L'abdomen est refermé et les animaux sont tués après 4 heures. La sécrétion gastrique est reprise et centrifugée à 1500 g pendant 15 minutes ; le volume du supernageant est mesuré et l'acidité est déterminée par titration automatique d'hydroxyde de sodium 0,05N. Un groupe de contrôle est inclus pour chaque expérimentation.

La dose nécessaire pour l'inhibition à 50 % (ED₅₀) de la sécrétion acide (X concentration d'acide chlorhydrique en volume) est calculée en utilisant la méthode décrite par Litchfield et Wilcoxon [J.T. Litchfield, F.A. Wilcoxon, J. Pharmacol., 96, 99 (1949)]. Les résultats obtenus pour les composés représentatifs de l'invention sont donnés dans le Tableau 3, et mis en comparaison avec les composés de référence cliniquement utilisés : Pirenzépine (anti-cholinergique), Ranitidine (antagoniste des récepteurs histaminergiques H₂) et Oméprazole (inhibiteur de la pompe à proton).

**Tableau 3**

| **Composé No.** | **ED**_{**50**} **(mg/kg i.d.)** |
|---|---|
| 1 | 3,7 |
| 2 | 3,6 |
| 18 | 3,2 |
| 20 | 3,1 |
| Pirenzépine | 14 |
| Ranitidine | 22 |
| Oméprazole | 4,6 |

### 3) Activité anti-émétique des composés de l'invention - Inhibition des vomissements induits par le cis-platine :

L'inhibition des vomissements induits par le cis-platine est évaluée chez le furet en utilisant une modification du protocole décrit par Barnes et ses collaborateurs [J.M. Barnes, N.M. Barnes, B. Costall, R.J. Naylor, F.D. Tattersall, Neuropharmacology, 27(8), 783 (1988)]. Ainsi, des furets albinos ou des furets-putois des deux sexes, pesant entre 0,5 kg et 1,5 kg sont anesthésiés avec du pentobarbitone (30-40 mg/kg i.p.) et une canule en polythène est insérée dans la veine jugulaire gauche. Deux jours plus tard, les composés testés sont administrés i.v. 30 minutes avant le cis-platine (30 mg/kg i.v.). Les animaux sont observés pendant 6 heures et le nombre de vomissements est enregistré. Un groupe contrôle est inclus pour chaque expérimentation.

Les résultats des composés représentatifs de l'invention sont donnés dans le Tableau 4 et mis en comparaison avec les composés-références cliniquement utilisés Ondansetron (antagoniste 5-HT₃) et Métoclopramide (antagoniste D₂).

**Tableau 4**

| **Composé No.** | **Dose (mg/kg i.v.)** | **% d'inhibition** |
|---|---|---|
| 1 | 1,0 | 40 |
| 18 | 1,0 | 62 |
| Ondansetron | 0,5 | 62 |
| Métoclopramide | 1 | 15 |

### 4) Effet des produits de l'invention sur le transit intestinal :

Un composé de l'invention a été testé pour déterminer son effet sur le transit intestinal, selon la méthode décrite par A.F. Green [Br. J. Pharmacol. , 14, 26-34 (1959)]. A des rats mâles Sprague Dawley pesant approximativement 200 g préalablement mis à jeun, on administre (p.o.) un repas consistant en du charbon (10 g) et de la gomme arabique (2,5 g) dissous dans 100 ml d'eau distillée. Les animaux sont sacrifiés après 15 minutes et la durée du transit du charbon est mesurée.

Le produit à tester à été administré (s.c.) 30 minutes avant l'administration du charbon. Les résultats obenus avec le composé No. 18 sont indiqués dans le Tableau 5.

**Tableau 5**

| **Composé No.** | **Dose (mg/kg s.c.)** | **Durée du transit du charbon par rapport aux témoins** |
|---|---|---|
| 18 | 0.3 | + 16 % |
| | 1,0 | +20% |

### 5) Effet des produits de l'invention sur la vidange gastrique :

Un composé de l'invention a été testé pour son effet sur la vidange gastrique selon la méthode décrite par C. Scarpignato et al. [Arch. Int. Pharmacodyn., 246, 286-294 (1980)]. Ainsi, 1,5 ml d'une solution à 1,5 % de méthylcellulose contenant du rouge phénol (0,5 mg/ml) a été administrée oralement à des rats Sprague Dawley pesant approximativement 200 g et mis préalablement à jeun. Les animaux sont sacrifiés après 15 minutes et les pylores et les oesophages sont bridés. Les contenus des estomacs sont collectés dans un becher en teflon contenant une solution de soude (100 ml à 0,1 N) et le mélange est homogénéisé. Les protéines sont précipitées par addition de 0,5 ml d'une solution aqueuse d'acide trifluoroacétique à 20 % à un aliquot de 5 ml de l'homogénat. Après centrifugation, la solution surnageante est séparée et ajoutée à 4,5 ml d'une solution aqueuse de soude à 0,5 N et le contenu en rouge phénol est déterminé en utilisant un spectrophotomètre à une longueur d'onde de 560 nM.

Le composé à tester est administré (s.c.) 15 minutes avec l'administration du rouge phénol.

Les résultats obtenus avec le composé No. 18 sont indiqués au Tableau 6.

**Tableau 6**

| **Composé No.** | **Dose (mg/kg s.c.)** | **Effet sur la vidange gastrique par rapport aux témoins** |
|---|---|---|
| 18 | 0,3 | + 8 % |
| | 1,0 | + 18 % |
| | 3,0 | + 26 % |

## Revendications

1. Les composés de formule générale **I** : 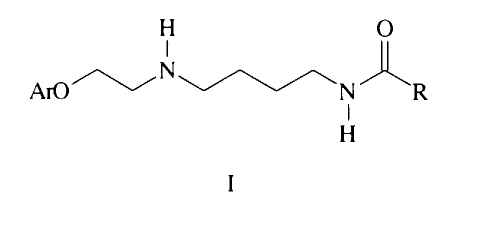 dans laquelle
Ar représente un phényle substitué par un ou plusieurs substituants choisis parmi les radicaux halogène, alkyle ayant de 1 à 6 atomes de carbone, alkoxy ayant de 1 à 6 atomes de carbone, cyano, nitro, hydroxy, C(O)NR¹R², C(O)NHOR³, NHC(O)R⁴, NHC(O)NHR⁵, CH₂NHC(O)NHR⁶, CH₂OR⁷, CH₂NHC(O)R⁸, CO₂R⁹, NHCO₂R¹⁰, C(O)R¹¹, SR¹² ;
dans lesquels R¹, R², R³, R⁴, R⁵, R⁶, R⁷, R⁸ sont, indépendamment, un atome d'hydrogène ou un alkyle ayant de 1 à 6 atomes de carbone et R⁹, R¹⁰, R¹¹, R¹² sont, indépendamment, un alkyle ayant de 1 à 6 atomes de carbone ;
R représente un radical hydrocarboné contenant de 1 à 10 atomes de carbone choisi parmi les radicaux alkyle, alkényle, alkynyle linéaires ou ramifiés, les radicaux cycloalkyle, cycloalkylalkyle ou alkylcycloalkyle, monocycliques ou polycycliques, saturés ou insaturés ;
le radical pyridyl ou isoquinolyl,
un phényle, éventuellement substitué par un ou plusieurs substituants choisis parmi les radicaux alkyle ayant de 1 à 6 atomes de carbone, alkoxy ayant de 1 à 6 atomes de carbone, halogène, hydroxy, nitro amino ou acylamino,
ainsi que les sels de ces produits.

2. Les composés de formule générale 1 telle que définie à la revendication 1, caractérisée en ce que Ar représente un radical phényle substitué par un substituant choisi parmi les radicaux hydroxy, méthoxy, -C(O)NHMé, -NHC(O)Mé, NHCO₂R'₁₀, R'₁₀ représentant un radical méthyle ou éthyle, -NHCONH₂, -C(O)NHOH et en ce que R représente un radical cyclopentyle, cyclohexyle, cycloheptyle, cyclopentylméthyle, adamantyle, tert-butyle, neopentyle, phényle, fluorophényle.

3. Les composés de formule générale 1 telle que définie à l'une des revendications 1 à 2 et répondant aux formules suivantes :
- la N[4-{2-(2-méthoxyphénoxy)ethyl}aminobutyl] benzamide ;
- la N[4-{2-(2-méthoxyphénoxy)ethyl}aminobutyl] adamantamide;
- la N[4-{2-(2-méthylamino carbonyle phénoxy)ethyl]amino}butyl]benzamide ;
- la N[4-{2-(2-hydroxyphénoxy)ethyl}aminobutyl]benzamide ;
- la N[4-{2-(2-méthoxyphénoxy)ethyl}aminobutyl]cyclohexylamide ;
- la N[4-{2-(2-méthoxyphénoxy)ethyl}aminobutyl]cycloheptylamide ;
- la N[4-{2-(2-méthoxyphénoxy)ethyl}aminobutyl]2-bicydo[2,2,1]heptylamide
ainsi que les sels de ces composés avec les acides minéraux ou organiques.

4. Procédé de préparation des produits de formule générale I telle que définie à la revendication 1, caractérisé en ce que
**A)** soit l'on fait agir un produit de formule II 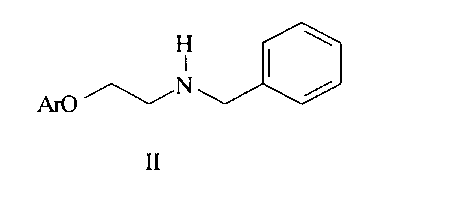 dans laquelle Ar a la signification indiquée à la revendication 1, avec un produit de formule III 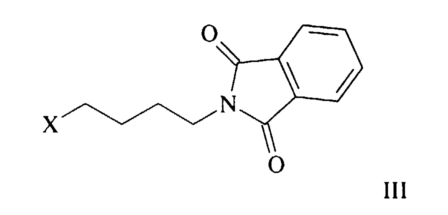 dans laquelle X représente un halogène ou un pseudo halogène pour obtenir un produit de formule IV 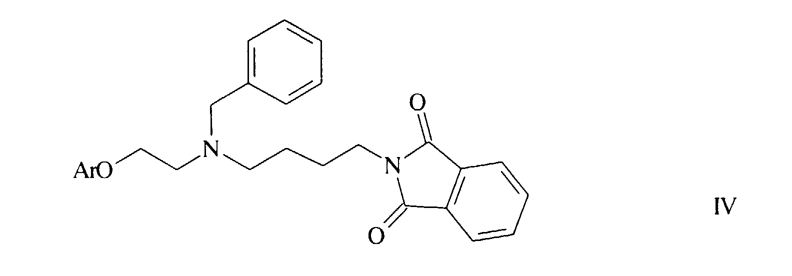 produit de formule IV que l'on soumet à une réaction d'élimination du groupement phtalimido pour obtenir un produit de formule V 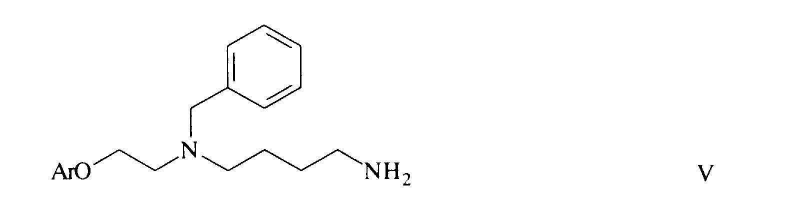 produit de formule V que l'on traite par réactif d'acylation dérivé de l'acide RCO₂H dans lequel R a la signification indiquée à la revendication 1, pour obtenir un produit de formule VI 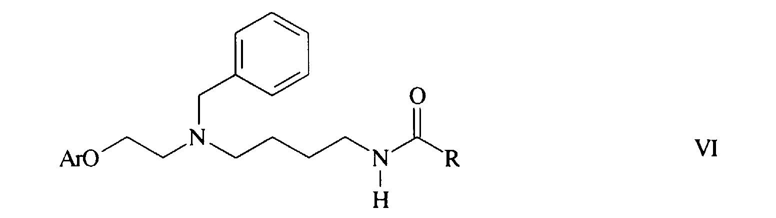
**B)** soit l'on fait agir un produit de formule VII 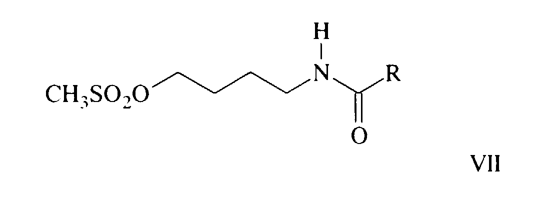 avec la N-benzyléthanolamine de formule 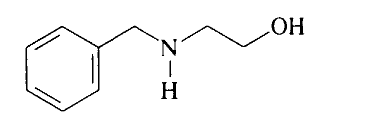 pour obtenir un produit de formule VIII 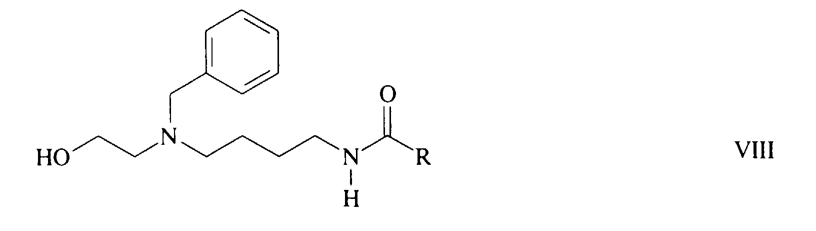 que l'on transforme en produit de formule IX 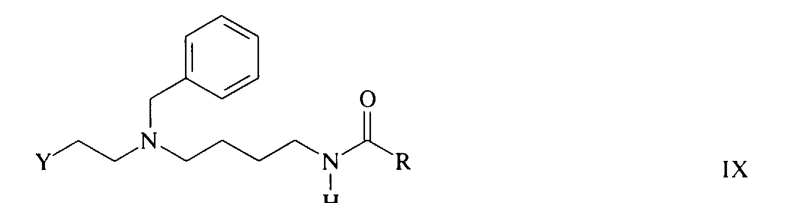 dans laquelle Y représente un radical halogène ou pseudo halogène, produit de formule IX que l'on transforme en produit de formule VI telle que définie précédemment en faisant agir un dérivé de phénol de formule ArOH,
produits de formule VI que l'on transforme en produit de formule I en clivant la fonction benzyle et produits de formule I que l'on transforme si désiré en sels d'acide par action de l'acide correspondant.

5. A titre de médicaments, les produits de formule I telle que définie à la revendication 1, ainsi que les sels d'addition avec les acides minéraux ou organiques pharmaceutiquement acceptables desdits produits de formule 1.

6. A titre de médicaments, les produits de formule I telle que définie à l'une des revendications 2 à 3, ainsi que les sels d'addition avec les acides minéraux ou organiques pharmaceutiquement acceptables desdits produits de formule 1.

7. Compositions pharmaceutiques contenant, à titre de principe actif, l'un au moins des médicaments tels que définis à l'une des revendications 5 ou 6.

8. Utilisation des produits de formule I telle que définie à l'une quelconque des revendications 1 à 3 pour la préparation de médicaments antiémétiques.

9. Utilisation des produits de formule I telle que définie à l'une quelconque des revendications 1 à 3 pour la préparation de médicaments destinés à réduire la sécrétion gastrique.

10. Utilisation des produits de formule I telle que définie à l'une quelconque des revendications 1 à 3 pour la préparation de médicaments destinés à accélérer la vidange gastrique.

11. Utilisation des produits de formule I telle que définie à l'une quelconque des revendications 1 à 3 pour la préparation de médicaments destinés à accélérer le transit intestinal.

12. Utilisation des produits de formule I telle que définie à l'une quelconque des revendications 1 à 3 pour la préparation de médicaments destinés à traiter l'anxiété, la dépression, les troubles du sommeil.

13. Utilisation des produits de formule I telle que définie à l'une quelconque des revendications 1 à 3 pour la préparation de médicaments destinés à traiter les maladies cardiovasculaires, notamment l'hypertension.

14. Les composés de formules IV, V, VI, VIII et IX telles que définies à la revendication 4.

## Patentansprüche

1. Verbindungen der allgemeinen Formel I: 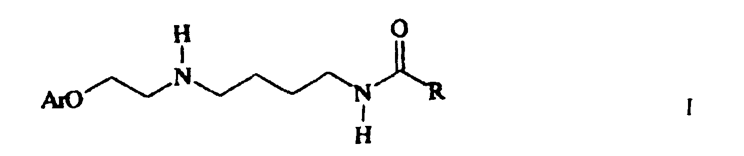 wobei
Ar in Phenyl repräsentiert, substituiert durch ein oder mehrere Ersatzmittel ausgewählt aus den Radikalen Halogen, Alkyl mit 1 bis 6 Kohlenstoffatomen, Alkoxy mit 1 bis 6 Kohlenstoffatomen, Cyano, Nitro, Hydroxy, C(O)NR¹R², C(O)NHOR³, NHC(O)R⁴, NHC(O)NHR⁵, CH₂NHC(O)NHR⁶, CH₂OR⁷, CH₂NHC(O)R⁸, CO₂R⁹, NHCO₂R¹⁰, C(O)R¹¹, SR¹²;
wobei R¹, R², R³, R⁴, R⁵, R⁶, R⁷, R⁸ unabhängig ein Wasserstoffatom oder ein Alkyl mit 1 bis 6 Kohlenstoffatomen und R⁹, R¹⁰, R¹¹, R¹² unabhängig ein Alkyl mit 1 bis 6 Kohlenstoffatomen sind;
R ein Kohlenwasserstoffradikal repräsentiert, das 1 bis 10 Kohlenstoffatome enthält, ausgewählt aus den Radikalen Alkyl, Alkenyl, lineares oder verzweigtes Alkynyl, den monzyklischen oder polyzyklischen, gesättigten oder ungesättigten Radikalen Cycloalkyl, Cycloalkylalkyl oder Alkylcycloalkyl;
das Radikal Pyridyl oder Isochinolyl,
ein Phenyl, bei Bedarf substituiert mit einem oder mehreren Ersatzmittel(n) ausgewählt aus den Radikalen Alkyl mit 1 bis 6 Kohlenstoffatomen, Alkoxy mit 1 bis 6 Kohlenstoffatomen, Halogen, Hydroxy, Nitro, Amino oder Acylamino,
sowie die Salze dieser Produkte.

2. Verbindungen der allgemeinen Formel I gemäß Definition in Anspruch 1, dadurch gekennzeichnet, daß Ar ein Phenylradikal repräsentiert, substitutiert durch ein Ersatzmittel ausgewählt aus den Radikalen Hydroxy, Methoxy, -C(O)NHM, -NHC(O)M, NHCO₂R'₁₀, wobei R'₁₀ ein Radikal Methyl oder Ethyl, -NHCONH₂, -C(O)NHOH repräsentiert, und dadurch, daß R ein Radikal Cyclopentyl, Cyclohexyl, Cycloheptyl, Cyclopentylmethyl, Adamantyl, tert.-Butyl, Neopentyl, Phenyl, Fluorphenyl repräsentiert.

3. Verbindungen der allgemeinen Formel I gemäß Definition in einem der Ansprüche 1 bis 2 und gemäß den folgenden Formeln:
- N[4-{2-(2-methoxyphenoxy)ethyl}aminobutyl]benzamid;
- N[4-{2-(2-methoxyphenoxy)ethyl}aminobutyl]adamantamid;
- N[4-{2-(2-methylamino-carbonylphenoxy)ethyl]amino}butyl]benzamid;
- N[4-{2-(2-hydroxyphenoxy)ethyl)aminobutyl]benzamid;
- N[4-{2-(2-methoxyphenoxy)ethyl}aminobutyl]cyclohexylamid;
- N[4-{2-(2-methoxyphenoxy)ethyl}aminobutyl]cycloheptylamid;
- N[4-{2-(2-methoxyphenoxy)ethyl)aminobutyl]2-bicyclo[2,2,1]heptylamid;
sowie die Salze dieser Verbindungen mit den mineralischen oder organischen Säuren.

4. Verfahren zur Herstellung von Produkten der allgemeinen Formel I gemäß Definition in Anspruch 1, dadurch gekennzeichnet, daß
A) entweder ein Produkt der Formel II: 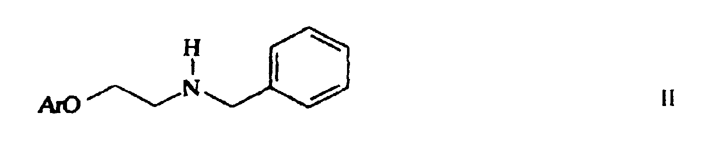 wobei Ar die in Anspruch 1 angegebene Bedeutung hat, mit einem Produkt der Formel III reagieren gelassen wird: 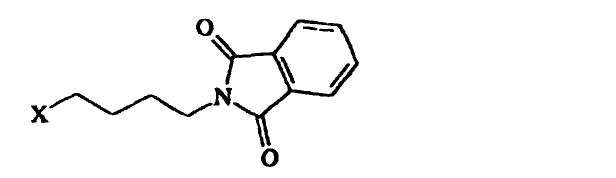 wobei X ein Halogen oder ein Pseudo-Halogen repräsentiert, um ein Produkt der Formel IV zu erhalten: 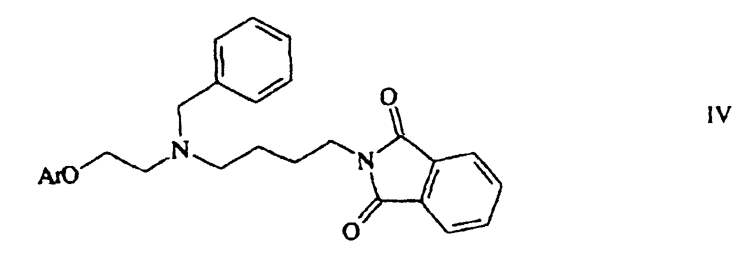 wobei das Produkt von Formel IV einer Phtalimio-Gruppen-Beseitigungsreaktion unterzogen wird, um ein Produkt der Formel V zu erhalten: 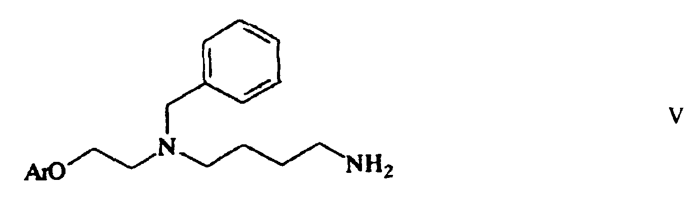 wobei das Produkt von Formel V einer Acylierungsreaktion unterzogen wird, deriviert von RCO₂H Säure, wobei R die in Anspruch 1 angegebene Bedeutung hat, um ein Produkt der Formel VI zu erhalten: 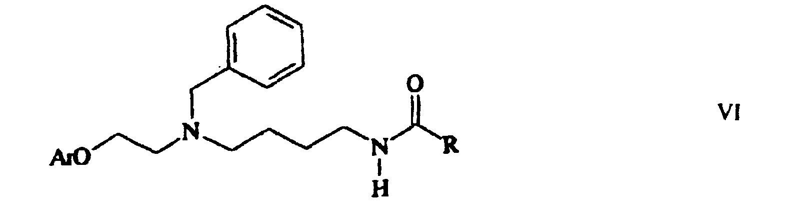
B) oder ein Produkt der Formel VII: 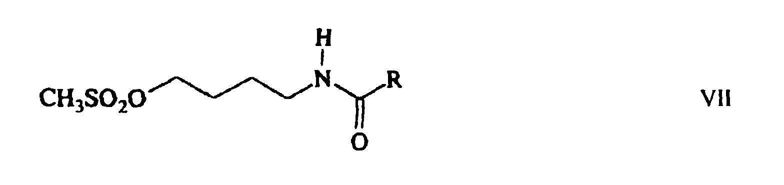 mit dem N-Benzylethanolamin der Formel reagieren gelassen wird, 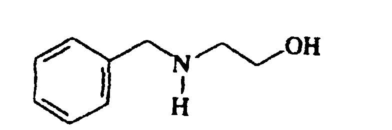 um ein Produkt der Formel Vlll zu erhalten: 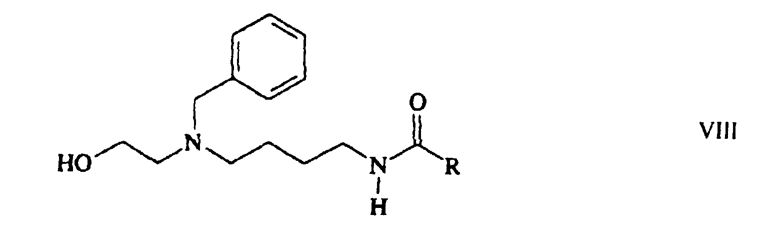 das in ein Produkt der Formel IX umgewandelt wird: 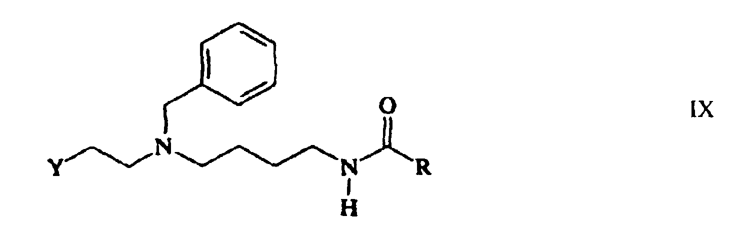 bei dem Y ein Halogen- oder Pseudo-Halogenradikal repräsentiert, wobei das Produkt der Formel IX in ein Produkt der Formel VI gemäß obiger Definition umgewandelt wird, indem es mit einem Phenolderivat der Formel ArOH reagieren gelassen wird,
wobei Produkte von Formel VI in ein Produkt der Formel 1 unter Abspaltung der Benzylfunktion umgewandelt wird, und wobei Produkte von Formel I bei Bedarf durch die Wirkung der entsprechenden Säure in Säuresalze umgewandelt werden.

5. Als Medikamente die Produkte von Formel I gemäß Definition in Anspruch 1, sowie die Additionssalze mit den mineralischen oder organischen, pharmazeutisch akzeptablen Säuren dieser Produkte von Formel I.

6. Als Medikamente die Produkte von Formel I gemäß Definition in einem der Ansprüche 2 bis 3, sowie die Additionssalze mit den mineralischen oder organischen, pharmazeutisch akzeptablen Säuren dieser Produkte von Formel I.

7. Pharmazeutische Verbindungen, die als Wirkstoff wenigstens eines der Medikamente gemäß Definition in einem der Ansprüche 5 bis 6 enthalten.

8. Verwendung von Produkten von Formel I gemäß Definition in einem der Ansprüche 1 bis 3 für die Herstellung von Antiemetika.

9. Verwendung von Produkten von Formel I gemäß Definition in einem der Ansprüche 1 bis 3 für die Herstellung von Medikamenten zur Reduzierung der Magen- und Darmsekretion.

10. Verwendung der Produkte von Formel I gemäß Definition in einem der Ansprüche 1 bis 3 für die Herstellung von Medikamenten zur Beschleunigung der Magen- und Darmentleerung.

11. Verwendung von Produkten von Formel I gemäß Definition in einem der Ansprüche 1 bis 3 für die Herstellung von Medikamenten zur Beschleunigung des Darmtransits.

12. Verwendung von Produkten von Formel I gemäß Definition in einem der Ansprüche 1 bis 3 für die Herstellung von Medikamenten zur Behandlung von Angst, Depressionen und Schlafstörungen.

13. Verwendung von Produkten von Formel I gemäß Definition in einem der Ansprüche 1 bis 3 für die Herstellung von Medikamenten für die Behandlung von Herz- und Kreislauferkrankungen, insbesondere Hypertonie.

14. Zusammensetzungen der Formeln IV, V, Vl, VIII und IX gemäß Definition in Anspruch 4.

## Claims

1. The compounds of general formula I: 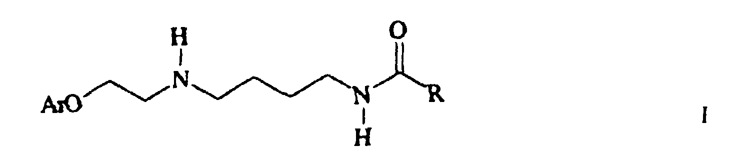 in which
Ar represents a phenyl substituted by one or more substituents chosen from the following radicals: halogen, alkyl having 1 to 6 carbon atoms, alkoxy having 1 to 6 carbon atoms, cyano, nitro, hydroxy, C(O)NR¹R², C(O)NHOR³, NHC(O)R⁴, NHC(O)NHR⁵, CH₂NHC(O)NHR⁶, CH₂OR⁷, CH₂NHC(O)R⁸, CO₂R⁹, NHCO₂R¹⁰, C(O)R¹¹, SR¹²;
in which R¹, R², R³, R⁴, R⁵, R⁶, R⁷, R⁸ are, independently, a hydrogen atom or an alkyl having 1 to 6 carbon atoms and R⁹, R¹⁰, R¹¹, R¹² are, independently, an alkyl having 1 to 6 carbon atoms;
R represents a hydrocarbon radical containing 1 to 10 carbon atoms chosen from linear or branched alkyl, alkenyl, alkynyl radicals, saturated or unsaturated, monocyclic or polycyclic cycloalkyl, cycloalkylalkyl or akylcycloalkyl radicals;
the pyridyl or isoquinolyl radical,
a phenyl, optionally substituted by one or more substituents chosen from the following radicals: alkyl having 1 to 6 carbon atoms, alkoxy having 1 to 6 carbon atoms, halogen, hydroxy, nitro amino or acylamino,
as well as the salts of these products.

2. The compounds of general formula I as defined in claim 1, characterized in that Ar represents a phenyl radical substituted by a substituent chosen from the following radicals: hydroxy, methoxy, -C(O)NHMe, -NHC(O)Me, NHCO₂R'₁₀, R'₁₀ representing a methyl or ethyl radical, -NHCONH₂, -C(O)NHOH and in that R represents a cyclopentyl, cyclohexyl, cycloheptyl, cyclopentylmethyl, adamantyl, tert-butyl, neopentyl, phenyl, fluorophenyl radical

3. The compounds of general formula I as defined in one of claims 1 to 2, and corresponding to the following formulae:
- N[4-{2-(2-methoxyphenoxy)ethyl}aminobutyl]benzamide;
- N[4-{2-(2-methoxyphenoxy)ethyl}aminobutyl]adamantamide;
- N[4-{2-(2-methylamino carbonyl phenoxy)ethyl]amino}butyl]benzamide;
- N[4-{2-(2-hydroxyphenoxy)ethyl}aminobutyl]benzamide;
- N[4-{2-(2-methoxyphenoxy)ethyl}aminobutyl]cyclohexylamide;
- N[4-{2-(2-methoxyphenoxy)ethyl}aminobutyl]cycloheptylamide;
- N[4-{2-(2-methoxyphenoxy)ethyl}aminobutyl]2-bicyclo[2,2,1]heptylamide
as well as the salts of these compounds with mineral or organic acids.

4. Preparation process for products of general formula I as defined in claim 1, characterized in that
A) either a product of formula II 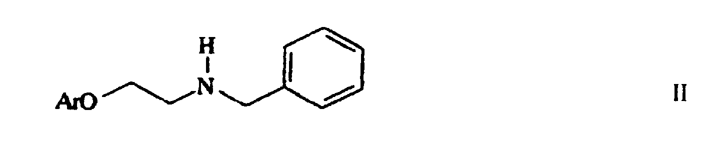 in which Ar has the meaning indicated in claim 1, is reacted with a product of formula Ill 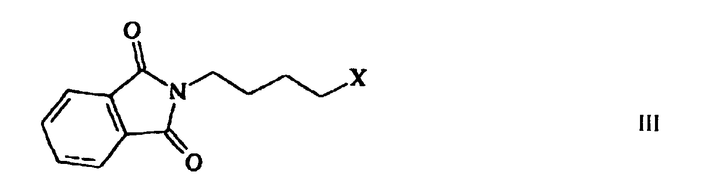 in which X represents a halogen or a pseudo halogen in order to obtain a product of formula IV 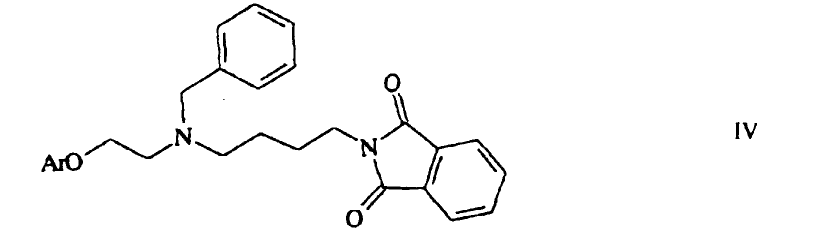 which product of formula IV is subjected to a reaction which eliminates the phthalimido group in order to obtain a product of formula V 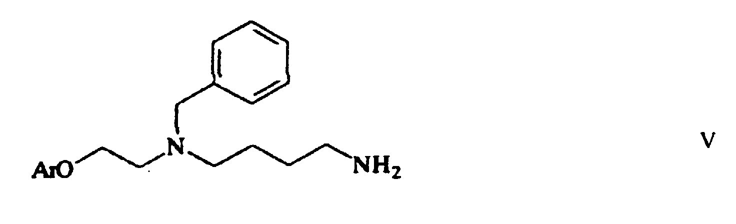 which product of formula V is treated with an acylation reagent derived from RCO₂H acid in which R has the meaning indicated in claim 1, in order to obtain a product of formula VI 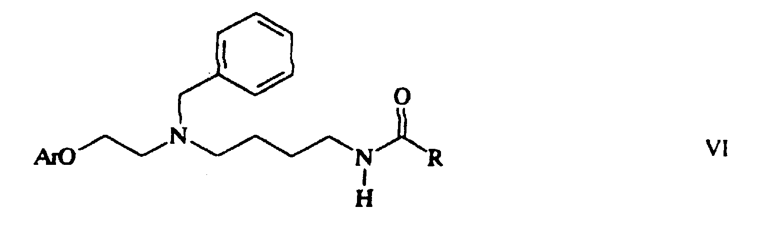
B) or a product of formula VII 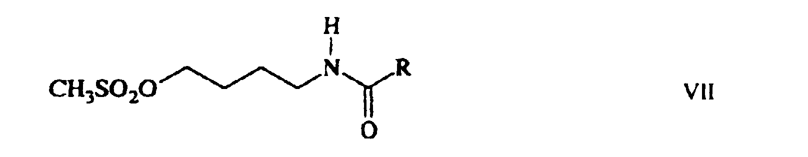 is reacted with the N-benzylethanolamine of formula 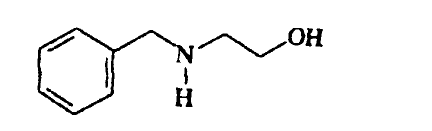 in order to obtain a product of formula VIII 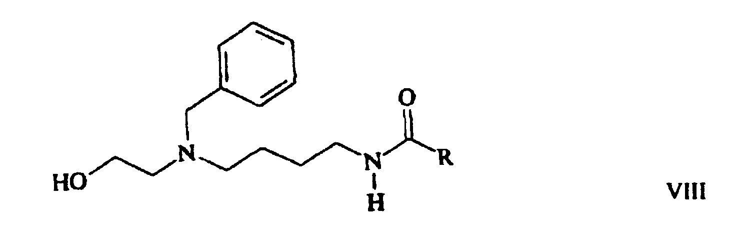 which is converted to a product of formula IX 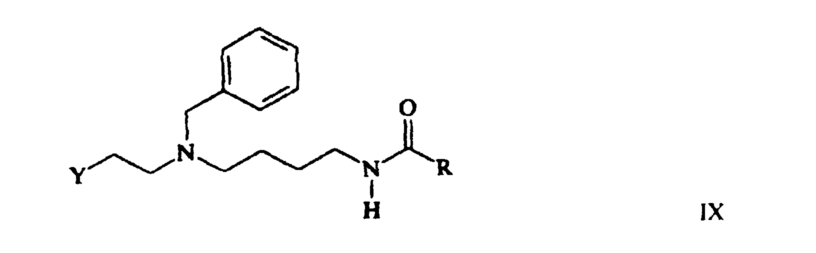 in which Y represents a halogen or pseudo halogen radical, which product of formula IX is converted to a product of formula VI as defined previously by reacting with a phenol derivative of formula ArOH,
which products of formula VI are converted to a product of formula I by cleaving the benzyl function and which products of formula I are converted, if desired, to acid salts by the action of the corresponding acid.

5. As medicaments, the products of formula I as defined in claim 1, as well as the addition salts with pharmaceutically acceptable mineral or organic acids of said products of formula I.

6. As medicaments, the products of formula I as defined in one of claims 2 to 3, as well as the addition salts with pharmaceutically acceptable mineral or organic acids of said products of formula I.

7. Pharmaceutical compositions containing at least one of the medicaments as defined in one of claims 5 or 6 as active ingredient.

8. Use of products of formula I as defined in any one of claims 1 to 3 for the preparation of antiemetic medicaments.

9. Use of products of formula I as defined in any one of claims 1 to 3 for the preparation of medicaments intended to reduce gastric secretion.

10. Use of products of formula I as defined in any one of claims 1 to 3 for the preparation of medicaments intended to accelerate gastric drainage.

11. Use of products of formula I as defined in any one of claims 1 to 3 for the preparation of medicaments intended to accelerate intestinal transit.

12. Use of products of formula I as defined in any one of claims 1 to 3 for the preparation of medicaments intended to treat anxiety, depression, sleep disorders.

13. Use of products of formula I as defined in any one of claims 1 to 3 for the preparation of medicaments intended to treat cardiovascular diseases, in particular hypertension.

14. The compounds of formulae IV, V, VI, VII and IX as defined in claim 4.
